# EUROPÄISCHE PATENTSCHRIFT

(11) **EP 2 220 991 B1**
(45) Veröffentlichungstag und Bekanntmachung des Hinweises auf die Patenterteilung: **21.09.2011**
(21) Anmeldenummer: 10153780.1
(22) Anmeldetag: 17.02.2010
(51) Int. Cl.: A61B 1/005, A61M 25/01

(54) **Instrument mit selbstentriegelndem Verstellrad**
Instrument with self-releasing adjustment wheel
Instrument doté d'une molette à déverrouillage automatique

(30) Priorität: 20.02.2009 DE 102009011434
(43) Veröffentlichungstag der Anmeldung: 25.08.2010
(73) Patentinhaber: Storz Endoskop Produktions GmbH, 78532 Tuttlingen (DE)
(72) Erfinder: Ettwein, Pierre, 8200 Schaffhausen (CH); Jäggli, Marcel, verstorben (CH); Klumpp, Martin, 78532 Tuttlingen (DE); Füllemann, Rolf, 8450 Andelfingen (CH)
(74) Vertreter: Witte, Weller & Partner

(56) Entgegenhaltungen:
- DE-A1- 19 650 721
- US-A1- 2002 165 484

## Beschreibung

Die Erfindung betrifft ein Instrument, mit einem proximalseitigen Kopfstück, mit einem zumindest im distalen Endbereich über einen Auslenkmechanismus auslenkbaren Schaft, mit einem Steuerelement, durch das Auslenkbewegungen des Schaftes durch Bewegen von Steuerseilen des Auslenkmechanismus steuerbar sind, und mit einer Sperre zum Sperren des Auslenkmechanismus.

Ein derartiges Instrument ist aus der DE 37 29 131 C1, der US 2002/165484 und der DE 19650721 bekannt.

Solche Instrumente haben zwischenzeitlich einen verbreiteten Einsatz gefunden.

Ein Einsatzbereich ist der medizinische Bereich. Die Instrumente weisen dazu zumindest am distalen Endbereich einen abwinkelbaren Schaftabschnitt auf.

Dadurch ist es möglich, den Schaft zunächst in ausgestrecktem Zustand in einem Körperhohlraum einzubringen. Dies kann beispielsweise durch einen in einem Körper eingebrachten Trokar bzw. dessen Trokarhülse erfolgen oder durch natürliche Körperöffnungen wie beispielsweise die Mundöffnung, den Anus oder über Harnröhren. In dem Hohlraum kann der Schaft seitlich ausgelenkt werden, so dass beispielsweise im Falle von Endoskopen, Beobachtungen abseits der geradlinigen Richtung vorgenommen werden können. Dabei können die Schäfte an sich starr oder auch flexibel aufgebaut werden, letztendlich ist entscheidend, dass der distale Endbereich ausgelenkt werden kann. Bei reinen Endoskopen erlaubt dies einen Rundumblick in der Körperhöhle. Sind am distalen Ende Operationsinstrumente, beispielsweise ein Nadelhalter angebracht, können auch an Stellen in dem Hohlraum, die durch ein geradliniges Instrument nicht erreichbar sind, Manipulationen z.B. Nähvorgänge durchgeführt werden.

Ein weiterer Einsatzbereich besteht insbesondere bei Endoskopen zur Inspektion von technischen Gerätschaften.

Ein Einsatzgebiet ist das Inspizieren von schwer zugänglichen Bauteilen, z.B. den hinteren Enden der Luftleitschaufeln von Turbinen von Flugzeugen. Durch auslenkbare Endoskope ist es möglich, solche schwer zugänglichen Stellen ohne Zerlegen der Turbinen zu inspizieren.

Weitere Einsatzgebiete sind beispielsweise der Motoren- oder der Karosseriebau, wobei insbesondere im letzten Fall Hohlraumversiegelungen auch in kompliziert geformten Karosseriehohlbauteilen inspiziert werden können.

Weitere technische Einsatzgebiete sind Inspektionen von Gebäuden oder Mauerwerken.

Der Anwendungsbereich für solche Instrumente mit auslenkbaren Schäften wird immer breiter.

Zur Bewerkstelligung der Auslenkung ist ein Auslenkmechanismus vorgesehen, der meist aus Steuerseilen besteht, die längs des Schaftes geführt werden und am Kopfstück mit einem Steuerelement in Verbindung stehen.

Soll beispielsweise das distale Ende in einer Ebene aus der geradlinigen Ausrichtung verschwenkt werden, sind zwei diametral gegenüberliegende Steuerseile vorgesehen. Zum Auslenken wird nun eines der beiden Steuerseile eingezogen und das diametral gegenüberliegende ausgeschoben, wodurch der flexible Endbereich des Schaftes in einer Ebene gekrümmt bzw. herausgebogen wird. Ein solches Steuerelement kann beispielsweise eine Spule oder eine Rolle aufweisen, an der die beiden Steuerseile befestigt sind. Ein Drehen der Spule durch das Steuerelement bewirkt in einer Richtung das Abspulen des einen Steuerseils und gleichzeitig ein Aufspulen des anderen Steuerseils, bei entgegengerichteter Drehrichtung läuft genau der umgekehrte Vorgang ab.

Dadurch ist es möglich, den auslenkbaren Bereich des Schaftes beidseits der Längsachse um nahezu 180° auszulenken.

Bei Weiterentwicklungen weist der Auslenkmechanismus zwei solche Steuerseilpaare auf, die um 90° drehversetzt angeordnet sind, so dass der auslenkbare Bereich des Schaftes in zwei orthogonal zueinander stehenden Ebenen ausgelenkt werden kann. Dabei hat sich etabliert, zwei unabhängig voneinander arbeitende Steuerelemente vorzusehen, wie das beispielsweise aus der DE 199 24 440 A1 bekannt ist. Eine Kombination beider Bewegungen erlaubt die Positionierung des distalen Endes auf einer Kugelfläche.

Im praktischen Einsatz wurde nun festgestellt, dass nach der Auslenkung auf den Schaft ein relativ starkes Rückstellmoment wirkt, das dazu neigt, den ausgelenkten Schaft wieder mehr oder weniger gerade auszurichten. Bei einem gebogenen flexiblen Schaft wird die äußere Hülle auf der einen Seite gestaucht, auf der anderen Seite gummituchartig gespannt. Daraus resultieren relativ starke Rückstellkräfte. Bestehen die Steuerseile aus relativ steifen Drähten, entstehen beim Auslenken, also Biegen der Drähte, ebenfalls Rückstellkräfte.

Um ein ungewolltes Rückstellen des ausgelenkten gebogenen Schaftes zu vermeiden, wurden Sperren entwickelt, die den ausgelenkten Schaft in einer bestimmten ausgelenkten Stellung vor Rückkehr sperren.

Dadurch wurde es notwendig zusätzlich zu dem Auslenkmechanismus noch einen Sperrmechanismus bereitzustellen, über den dann der Schaft in einer jeweils ausgelenkten Stellung vor Rückkehr gesperrt werden kann.

Bei der eingangs genannten DE 37 29 131 C1 wird dies durch einen kombinierten Brems- und Steuerhebel erzielt. Der Hebel, dessen mittige Drehachse mit einer Spule verbunden ist, auf der die Steuerseile auf- oder abwickelbar sind, stellt das Steuerelement des Auslenkmechanismus dar. Wurde nun mittels des Steuerhebels der Schaft in eine bestimmte Stellung ausgelenkt, muss der Hebel, in eine andere Richtung bewegt werden, um dadurch einen Bremsmechanismus zu betätigen, der den ausgelenkten Bereich vor Rückstellung hindern soll, also diese Bewegung sperren soll. Eine gewisse Hin- und Herbewegung zur Feinkorrektur ist aber nach wie vor möglich.

Soll der Schaft in eine andere Auslenkposition bewegt oder wieder gerade ausgerichtet werden, muss über den kombinierten Brems- und Steuerhebel durch eine Bewegung in einer Richtung zunächst der Bremsmechanismus gelöst werden und dann, in einer anderen Bewegungsrichtung die Steuerseile des Auslenkmechanismus bewegt werden.

Dies ist dahingehend nachteilig, dass mit ein und demselben Steuerelement unterschiedliche Bewegungsrichtungen und Manipulationen durchgeführt werden müssen, um die unterschiedlichen Vorgänge, nämlich einmal Betätigen des Auslenkmechanismus und zum andern Betätigen des Bremsmechanismus durchführen zu können.

In einer Grundstellung ist der Bremsmechanismus frei und durch den Hebel kann nun der Auslenkmechanismus betätigt werden. Anschließend muss der Hebel zielgerichtet in eine andere Richtung bewegt werden, um den Bremsmechanismus zu aktivieren.

Dies ist ergonomisch ungünstig, erfordert eine hohe Aufmerksamkeit und beinhaltet die Gefahr, falls der Bremsmechanismus nicht zutreffend betätigt ist, dass sich der ausgelenkte Schaft verstellt.

Die Bedienungsperson erkennt auch nicht unmittelbar, ob sich der Hebel in einer Stellung befindet, in der er drehbar ist, um den Auslenkmechanismus zu betätigen, oder ob er sich schon in der Bremsfunktion befindet, denn er muss dazu nur wenige Millimeter aus der Drehposition angehoben werden.

Das beinhaltet das Problem, dass, wenn versehentlich auf den Hebel gedrückt wird, sich die Bremsfunktion löst und der ausgelenkte Schaft sich bewegt.

Dies ist insbesondere im medizinischen Bereich fatal, wenn beispielsweise mit dem ausgelenkten Ende gerade ein chirurgischer Eingriff durchgeführt wird.

Es ist daher Aufgabe der vorliegenden Erfindung, hier Abhilfe zu schaffen und ein Instrument der eingangs genannten Art dahingehend weiterzuentwickeln, dass die Steuerung und insbesondere das Sperren und Entsperren betriebssicher durchführbar ist, insbesondere bei einer Einhandbedienung.

Erfindungsgemäß wird die Aufgabe dadurch gelöst, dass die Sperre einen Rastmechanismus aufweist, der derart mit dem Steuerelement verbunden ist, dass der Rastmechanismus, ohne Betätigung des Steuerelements, zwangsläufig in eine sperrende Stellung läuft, und dass eine Bewegung des Steuerelements in eine Richtung zunächst eine Öffnung des Rastmechanismus bewirkt und erst dann eine Auslenkung des Schaftes ermöglicht, und, dass eine Freigabe des Steuerelements in irgendeiner Stellung des Auslenkmechanismus, eine zwangsläufige Sperrung durch den Rastmechanismus in dieser Stellung verursacht.

Diese Maßnahmen haben nunmehr mehrere Vorteile.

Durch Vorsehen eines Rastmechanismus ist ein mechanisch einfaches und wirksames Mittel geschaffen, um den Schaft vor ungewünschten Auslenkbewegungen zu sperren.

Dieser Rastmechanismus wird zwangsläufig, d.h. automatisch in die sperrende Stellung gebracht, wenn das Steuerelement nicht bewegt wird oder nach einer Bewegung freigegeben wird. Der Grund- oder Normalzustand ist so, dass der Rastmechanismus sich in seiner sperrenden Stellung befindet.

Dies hat den Vorteil, dass beispielsweise zur Lagerung, zum Transport oder zur Vorbereitung von Handhabungsmaßnahmen sich der Schaft in einer klar definierten Ausrichtung befindet, aus der er sich nicht ohne Lösen des Rastmechanismus herausbewegen kann. Man kann also den flexiblen auslenkbaren Bereich in dieser Raststellung wie einen starren Schaft handhaben.

Erst wenn das Steuerelement betätigt wird, nämlich bewegt wird, erfolgt zunächst eine Öffnung des Rastmechanismus und bei weiterer Bewegung ist dann eine Auslenkung des Schafts über den Auslenkmechanismus möglich. Wird das Steuerelement freigegeben und zwar in irgendeiner Stellung des Auslenkmechanismus erfolgt eine zwangläufige unmittelbare und sofortige Bewegung in die sperrende Stellung. Die Handhabungsperson muss das Steuerelement nicht in eine wie auch immer gestaltete andere Position bewegen, sondern gibt dieses lediglich frei, wodurch der Rastmechanismus sofort wieder in die sperrende Stellung einrastet. Letztendlich muss die Handhabungsperson das Steuerelement nur in eine Richtung bewegen, um zunächst den Rastmechanismus zu lösen und dann anschließend die Auslenkbewegungen durchzuführen. Nach Freigabe wird sofort der Rastmechanismus wieder in seine sperrende Stellung gebracht, ohne dass weitere Auslenkbewegungen möglich sind.

Die Handhabungsperson muss also nicht mehr wissen oder gar erforschen, ob sich das Steuerelement in einem Zustand befindet, in dem es sperrt oder eine Auslenkbewegung ermöglicht, denn es befindet sich automatisch und zwangsläufig immer im sperrenden Zustand. Bei jeder Bewegung des Steuerelementes wird immer zunächst die Sperrung gelöst und erst danach kann die Auslenkbewegung erfolgen, sei dies nun eine Bewegung um einen gradlinigen Schaft auszulenken oder einen vormals ausgelenkten Schaft wieder in die geradlinige Stellung zu verbringen.

In einer weiteren Ausgestaltung der Erfindung weist der Rastmechanismus eine Reihe an Zähnen auf, in die eine bewegbare Klinke zwangsläufig sperrend einfahrbar ist, die vom Steuerelement aus dem sperrenden Eingriff heraus bewegbar ist.

Diese Maßnahme hat den Vorteil, dass durch mechanisch einfache und dennoch robuste Mittel diese Zwangssteuerung durchgeführt werden kann.

In einer weiteren Ausgestaltung der Erfindung ist die Klinke federelastisch in Richtung der Reihe an Zähnen vorgespannt.

Diese Maßnahme hat den Vorteil, dass die Klinke permanent in Richtung des sperrenden Eingriffes mit der Reihe an Rastzähnen vorgespannt ist, sich somit in jedwedem Zustand in diese Richtung bewegen möchte. Dies kann nun dadurch geschehen, dass die Klinke durch die Kraft einer Feder beaufschlagt wird, oder dass die Klinke selbst aus federelastischem Material oder entsprechend geformt und in sich selbst vorgespannt ist.

Dies trägt zu einem einfachen mechanischen Aufbau und einer auf Dauer betriebssicheren Handhabung bei.

In einer weiteren Ausgestaltung der Erfindung sind die Reihe an Zähnen und die Klinke derart geformt, dass eine Bewegung der Klinke längs der Reihe gesperrt ist, und erst eine Bewegung der Klinke aus der Reihe heraus eine Bewegung ermöglicht.

Diese Maßnahme hat den Vorteil, dass in eingerastetem Zustand eine Relativbewegung längs der Reihe an Zähnen wirkungsvoll gesperrt ist. Erst wenn die Klinke aus der Reihe an Zähnen herausbewegt ist, kann eine Bewegung des Auslenkmechanismus erfolgen. Insbesondere in Zusammenhang mit der zuvor genannten Maßnahme der vorgespannten Klinke sind durch mechanisch einfache und robuste Bauteile die Voraussetzungen geschaffen, zum einen ein Einrasten oder eine Verriegelung sicherzustellen, die automatisch immer dann geschlossen wird, wenn die Klinke freigegeben ist, zum anderen schon kleinste Bewegungen des Steuerelements ausreichen, um die Klinke aus dem sperrenden Eingriff mit der Reihe an Zähnen herauszubewegen.

In einer weiteren Ausgestaltung der Erfindung ist die Reihe an Zähnen und die Klinke derart geformt, dass unter Überwindung einer vorgegebenen Gegenkraft, die Klinke längs der Reihe an Zähnen bewegbar ist.

Durch Anpassung der Formgebung zwischen Zähnen und Klinke und der entsprechenden Anpresskraft der Klinke an die Reihe an Zähnen kann zum einen sichergestellt werden, dass die Verrastung längs der Reihe der Zähne erheblichen Kräften widersteht, und erst bei Überschreitung einer bestimmten Gegenkraft die Klinke ausrastet. Dann ist es möglich, dass die Klinke die Reihe an Zähnen überläuft. Es ist aber zu jeder Zeit sichergestellt, dass, sofern das Steuerelement losgelassen wird, die Klinke sofort in die nächst mögliche Lücke zwischen zwei Zähnen automatisch eintritt und dadurch wieder den sperrenden Eingriff bewerkstelligt.

In einer weiteren Ausgestaltung der Erfindung ist die Reihe an Zähne und die Klinke derart geformt, dass eine Bewegung der Klinke in einer Richtung der Reihe gesperrt und in der entgegengesetzten Richtung unter Überwindung einer Gegenkraft möglich ist.

Diese Möglichkeit hat den Vorteil, dass dadurch besonders sicher eine bidirektionale oder gegenläufige Auslenkung bzw. Steuerung möglich ist.

Stellt man sich einen Schaft in geradliniger Ausrichtung vor, so kann er mit einem Seilzugpaar in einer Ebene beispielsweise nach links oder nach rechts ausgelenkt werden. Will man nun den Schaft nach links auslenken, so kann man durch die zuvor erwähnte Maßnahme erreichen, dass in dieser Richtung die Sperrung vollständig aufgehoben ist. In der entgegengesetzten Richtung kann die Zahnreihe überlaufen werden. Ordnet man jeder Bewegungsrichtung eine Reihe an Zähnen zu, so können diese von einer Klinke in einer Richtung überfahren werden, in der entgegengesetzten Richtung muss die Klinke erst von der Reihe abgehoben werden. Durch eine zweite Klinke mit entgegengesetzter Charakteristik sperrt diese in der Richtung, in der die andere Klinke die Zähne überfahren kann und umgekehrt.

In weiterer Ausgestaltung der Erfindung weist die Klinke einen asymmetrischen Klinkzahn auf, der die Bewegung der Klinke in einer Richtung durch Formschluss sperrt und in der anderen unter Überwindung einer Gegenkraft die Reihe an Zähnen überlaufen kann.

Diese Maßnahme hat den Vorteil, dass durch konstruktiv einfache und mechanisch robuste Maßnahmen die bidirektionale gerichtete Bewegung möglich ist.

In einer weiteren Ausgestaltung der Erfindung sind dazu zwei Klinken vorhanden, wobei eine erste Klinke in einer ersten Richtung längs der Reihe an Zähnen sperrt und eine zweite Klinke in einer zweiten, der ersten entgegengesetzten Richtung sperrt, wobei das Steuerelement bei Bewegen in die erste Richtung die erste Klinke aus dem sperrenden Eingriff löst und beim Bewegen in der zweiten Richtung die zweite Klinke löst.

Diese Maßnahme hat den Vorteil, dass sofern das Steuerelement nicht bewegt wird, beide Klinken im sperrenden Eingriff stehen und somit in beide Bewegungsrichtungen sperren.

Wird nun das Steuerelement in einer Richtung bewegt, bringt es zunächst diejenige Klinke aus der sperrenden Stellung, die diese Bewegungsrichtung sperrt. Die andere Klinke kann dabei, unter Überwindung einer gewissen Anpresskraft, die Reihe an Zähnen überlaufen.

Wird das Steuerelement in die entgegengesetzte Richtung bewegt, hebt es zunächst die in dieser Richtung sperrende Klinke aus der Reihe an Zähnen heraus, so dass die Bewegung in diese Richtung ermöglicht ist, wobei dann die andere Klinke eben wieder unter Überwindung einer gewissen Anpresskraft über die Reihe an Zähnen läuft.

Hier ist durch konstruktiv einfache Maßnahmen sichergestellt, dass in der Richtung, in der eine Klinke sperrt, diese zunächst vom Steuerelement, wenn es in diese Richtung bewegt wird, aus dem sperrenden Eingriff abgehoben wird, die andere Klinke aber noch unter einem gewissen Anpressdruck die Reihe an Zähnen in dieser Richtung überlaufen kann. Somit steht immer eine der Klinken unter einem gewissen Anpressdruck mit der Reihe an Zähnen in Verbindung, so dass sichergestellt ist, dass unmittelbar nach dem das Steuerelement freigegeben wird, diese Klinke in den rastenden Eingriff tritt.

In einer weiteren Ausgestaltung der Erfindung ist die Reihe an Zähnen als Ritzel ausgebildet, um das die Klinken als schwenkbare einarmige Hebel angeordnet sind.

Diese Maßnahme hat den Vorteil, dass damit eine sehr kompakt bauende Konstruktion möglich ist. Das Ritzel stellt eine endlose Bahn an Zähnen zur Verfügung, um dessen Außenseite herum können dann entsprechend geformte oder eine entsprechend geeignete Anzahl an Klinken angeordnet werden.

In einer weiteren Ausgestaltung der Erfindung weist das Ritzel zwei Reihen an Zähnen auf, wobei die Zähne einer ersten Reihe mit der ersten Klinke und die Zähne einer zweiten Reihe mit der zweiten Klinke in Eingriff bringbar sind.

Diese Maßnahmen haben mehrere Vorteile. Zunächst einmal kann jede Reihe an Zähnen optimal auf die Klinke abgestimmt sein, mit der sie in Eingriff steht. Meistens ist das Sperrelement der Klinke ein Klinkenzahn, der mit den Zähnen des Ritzels in Wirkverbindung steht, und zwar derart, dass er in der einen Drehrichtung des Ritzels absolut sicher sperrt, in der entgegengesetzten Richtung aber die Reihe an Zähnen überlaufen kann. Dadurch, dass nun für jede Klinke eine eigene Reihe an Zähnen vorhanden ist, kann die Geometrie der Zähne der einen Reihe optimal an die Geometrie eines Klinkenzahnes angepasst werden. Das führt dazu, dass das Spiel beim Auslösen des Rastmechanismus wesentlich verringert werden kann, da die Zähne der einen Reihe nur auf den Klinkenzahn einer der Klinken abgestimmt sein muss. Dementsprechend kann dann die andere Reihe selbstverständlich optimal an den Klinkenzahn der anderen Klinke angepasst sein. Im Ergebnis führt das dann dazu, dass in beide Drehrichtungen gesehen ein geringes Spiel beim Auslösen des Rastmechanismus vorhanden ist. Dies eröffnet die Möglichkeit, die Zähne entsprechend asymmetrisch auszubilden und auszurichten, so dass auch bei kleinster Bauweise in einer Drehrichtung eine optimale Sperrwirkung erzielt wird, in der entgegengesetzten Richtung ein einfaches Überlaufen dieser Zähne durch die Klinke möglich ist.

In einer weiteren Ausgestaltung der Erfindung ist das Ritzel aus zwei übereinander gesetzten Ritzelscheiben aufgebaut, die jeweils eine Reihe an entgegengesetzt gerichteten Zähnen aufweisen.

Diese Maßnahme hat den Vorteil, dass die beiden Ritzelscheiben zunächst einfach als einzelne Bauteile herstellbar sind und dann zu dem Ritzel mit zwei Reihen zusammengesetzt werden können. Es ist dabei möglich, zwei identische Ritzelscheiben herzustellen und diese umgekehrt aufeinander zu setzen. Es ist auch möglich, an einer Ritzelscheibe ein Montagemerkmal vorzusehen, über das es dann lagegerecht mit dem anderen Ritzel zusammengesetzt werden kann. Dieses Montagemerkmal kann beispielsweise ein Flansch sein, der von einer Ritzelscheibe hoch steht, auf den die andere Ritzelscheibe aufgeschoben werden kann. Hier eröffnen sich zahlreiche Montagemöglichkeiten.

In einer weiteren Ausgestaltung der Erfindung ist das Steuerelement als Drehrad ausgebildet, das um das ortsfest angeordnete Ritzel drehbar ist.

Diese Maßnahme hat den Vorteil, dass das Steuerelement ergonomisch sehr günstig ausgebildet und zu erfassen ist, und dass über das ortsfeste Ritzel die Sperrkräfte auf das Instrument verteilt abgelenkt oder überführt werden können.

In einer weiteren Ausgestaltung der Erfindung ist am Drehrad ein Mitnehmer angeordnet, der in jeder Drehstellung mit einer der Klinken in Eingriff kommt und diese dabei aus dem sperrenden Eingriff bringt und so hält, solange das Drehrad die dazu erforderliche Kraft auf die Klinke ausübt.

Diese Maßnahme hat den Vorteil, dass der Auslenkmechanismus solange bewegt oder auch ggf. noch korrigiert werden kann, solange diese sperrende Klinke durch das Drehrad außer Eingriff gehalten wird. Dieser Zustand fängt ja damit an, dass man das Drehrad ergreift und bewegt. Hat man eine bestimmte Auslenkstellung erreicht, kann man weiterhin noch das Drehrad in der Hand halten, also gegen die Rückstellkraft des ausgelenkten Schafts, um ggf. die exakte Auslenkstellung zu überprüfen. Nach Freigabe des Drehrades rastet dann die Klinke sofort ein und sperrt eine weitere Bewegung. Wählt man einen großen Auslenkhebel, also lange Klinken, und einen kleinen Zahneingriffhebel wird der Mechanismus besonders leichtgängig. Außerdem wird ein Verhaken vermieden.

In einer weiteren Ausgestaltung der Erfindung wird die ausgerastete Klinke vom Mitnehmer gegen einen Anschlag an einer Mitnehmerscheibe gedrückt, die kraftschlüssig mit dem Auslenkmechanismus in Verbindung steht.

Diese Maßnahme hat den Vorteil, dass die Drehbewegung des Drehrades zunächst dazu herangezogen wird, die sperrende Klinke aus dem sperrenden Eingriff zu bringen, ohne dass dadurch schon eine Auslenkung des Schaftes erfolgt. Erst nachdem der Mitnehmer die Klinke gegen einen Anschlag an einer Mitnehmerscheibe drückt, wird diese gedreht und dann wird die Drehbewegung auf den Auslenkmechanismus übertragen. In diesem Zustand kann die Handhabungsperson das Maß der Drehung bzw. Auslenkung anhand des Maßes verfolgen bzw. abschätzen, um das das Drehrad verschwenkt wird. Dann kann er ggf. auch ohne direkte visuelle Kontrolle feststellen, wieweit denn das auslenkbare Ende ausgelenkt ist.

In einer weiteren Ausgestaltung der Erfindung sind bei einem Instrument, dessen Schaft unabhängig in zwei Ebenen auslenkbar ist, zwei derartig ausgestaltete Auslenkmechanismen vorhanden.

Diese Maßnahme hat den Vorteil, dass bei solchen Geräten in beiden Auslenkebenen entsprechend dem Grundgedanken der Erfindung diese Bewegungen sicher gezielt und ergonomisch einfach durchgeführt werden können.

In einer weiteren Ausgestaltung der Erfindung sind bei dieser Variante zwei aufeinandergesetzte Drehräder vorgesehen, die mechanisch aneinander gehalten sind, jedoch eine Betätigung jedes der Drehräder unabhängig vom anderen ermöglichen.

Diese Maßnahme hat den Vorteil, dass durch die beiden Drehräder auf gleiche Art und Weise beispielsweise die Auslenkbewegungen links und rechts bzw. nach oben und unten durchgeführt werden können.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger Ausführungsbeispiele im Zusammenhang mit den beiliegenden Zeichnungen näher beschrieben und erläutert. Es zeigen:
Fig. 1 eine perspektivische Ansicht eines Instrumentes mit einem Schaft, dessen distaler Endbereich auslenkbar ist,
Fig. 2 einen Schnitt längs der Linie II-II in Fig. 1,
Fig. 3 eine stark vergrößerte seitliche Draufsicht auf ein Drehrad des Instrumentes von Fig. 1.
Fig. 4 ein Bauteil, nämlich eine Klinke des in Fig. 3 dargestellten Drehrades,
Fig. 5 eine stark vergrößerte ausschnittsweise Ansicht eines Klinkenzahnes der Klinke,
Fig. 6 ausschnittsweise eine Schnittdarstellung der Klinke von Fig. 4, deren Klinkenzahn zwischen zwei Zähne einer Reihe an Zähnen eingerastet ist,
Fig. 7 eine der Fig. 6 vergleichbare Darstellung, bei der der Klinkenzahn einen Zahn der Reihe an Zähnen gerade überläuft,
Fig. 8 eine der Fig. 6 vergleichbare Darstellung eines anderen Ausführungsbeispieles für einen Klinkenzahn,
Fig. 9 eine den Fig. 6 und 8 vergleichbare Darstellung einer weiteren Ausführungsform eines Klinkenzahnes,
Fig. 10 eine der Fig. 3 vergleichbare Darstellung in einer Position, in der das Drehrad etwas im Uhrzeigersinn verdreht wurde und eine Klinke gerade aus der Reihe an Zähnen abgehoben ist,
Fig. 11 eine den Fig. 3 und 10 vergleichbare Stellung nach einer Drehung des Drehrades um 90° im Uhrzeigersinn,
Fig. 12 eine der Fig. 11 vergleichbare Darstellung, nach dem das Drehrad freigegeben wurde,
Fig. 13 eine der Darstellungen von Fig. 10 vergleichbare Darstellung, wobei sich das Drehrad aus der in Fig. 12 gezeigten Darstellung etwas entgegen dem Uhrzeigersinn bewegt hat,
Fig. 14 eine Seitenansicht des Drehrades von Fig. 3, teilweise im Schnitt,
Fig. 15 eine Schnittdarstellung, in der auf das von Fig. 14 dargestellte erste Drehrad ein weiteres Drehrad aufgesetzt ist,
Fig. 16 eine der Darstellung von Fig. 3 vergleichbare Draufsicht auf ein weiteres Ausführungsbeispiel mit einem Doppelritzel, und
Fig. 17 einen Schnitt längs der Linie XVII-XVII in Fig. 16.

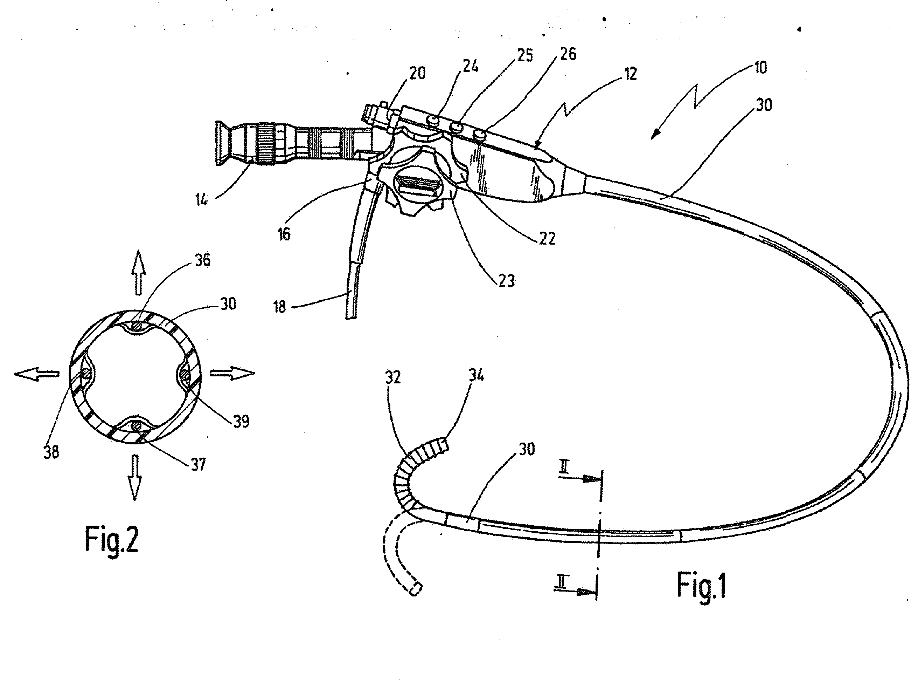

Ein in Fig. 1 und 2 dargestelltes flexibles Endoskop ist in seiner Gesamtheit mit der Bezugsziffer 10 bezeichnet. Das flexible Endoskop 10 ist ein Instrument, das im medizinischen Bereich Einsatz findet, beispielsweise zur Untersuchung des Verdauungsapparates von großen Tieren wie Rindern oder Pferden von rektal.

Dieses Instrument unterscheidet sich prinzipiell nicht von einem flexiblen Endoskop, das im technischen Bereich eingesetzt wird, beispielsweise zur Inspektion der Hinterseite von Turbinenschaufeln eines Turbinentriebwerks für ein Flugzeug.

Das Endoskop 10 weist ein Kopfstück 12 auf. Proximalseitig steht vom Kopfstück 12 ein Okular 14 vor. Ein seitlich vorstehender Anschluss 16 dient dazu, einen Leitungsstrang 18 anzuschließen, der Leitungen für Beleuchtung, Spülung, Insufflation, Absaugung oder dergleichen enthalten kann. Ein weiterer, sich etwa in Richtung des Okulars 14 erstreckender Anschluss 20 ist dazu vorgesehen, dass über diesen andere Instrumente, beispielsweise Zangen, Schlingen oder dergleichen in das Instrument eingeschoben werden können. Seitlich am Kopfstück 12 sind zwei Steuerelemente 22 und 23 in Form von Drehrädern angeordnet, mit denen die Blickrichtung des Endoskopes, wie nachfolgend noch erläutert, verstellt werden kann. Im Bereich des Kopfstückes 12 sind ferner Schalter 24, 25, 26 angeordnet, über die verschiedene Funktionen, wie beispielsweise Saugen, Spülen oder dergleichen, gesteuert werden können.

Vom Kopfstück 12 erstreckt sich ein lang erstreckter flexibler Schaft 30 fort, der im dargestellten Ausführungsbeispiel die Länge von etwa 1 m aufweist. Der Schaft 30 ist aus einem flexiblen vielschichtigen Aufbau hergestellt, der ein Krümmen und Biegen des Schaftes 30, wie in Fig. 1 dargestellt, ermöglicht. Ein distaler Endbereich 32 weist gegenüber dem Schaft 30 eine nochmals erhöhte Biegsamkeit dahingehend auf, dass der distale Endbereich 32 zusätzlich noch mehr als um 180° halbkreisförmig ausgelenkt werden kann. Dies ist zum einen in einer Ebene möglich, wie das in Fig. 1 dargestellt ist, also in der Zeichenebene. Darüber hinaus ist der distale Endbereich 32 auch senkrecht zur Ebene, also nach oben und nach unten von dieser Ebene weg auslenkbar. Beide Auslenkbewegungen können gleichzeitig durchgeführt werden, dadurch bewegt sich das distale Abschlussstück 34 (oder auch Schnabel genannt) auf einer Kugelfläche.

Wie aus der Schnittdarstellung von Fig. 2 zu erkennen, sind im Innern des Schafts 30 jeweils zwei Paare an Steuerseilen 36 und 37 bzw. 38 und 39 angeordnet. Diese bestehen dabei aus einem relativ steifen Draht. Das Paar an Steuerseilen 36 und 37 steht mit dem Steuerelement 22 am Kopfstück 12 in Verbindung. Wird das Steuerelement 22 beispielsweise im Uhrzeigersinn verdreht, wird das Steuerseil 36 auf eine mit dem Steuerelement 22 in Wirkverbindung stehende Rolle aufgewickelt und gleichzeitig wird dabei das Steuerseil 37 abgewickelt. Statt einer Rolle kann auch ein Kettentrieb mit Zahnrad vorhanden sein. Dadurch erfolgt eine Auslenkung des distalen Endbereiches 32 nach oben. Wird das Steuerelement 22 in die entgegengesetzte Richtung gedreht, wird das Steuerseil 37 aufgewickelt und das Steuerseil 36 abgewickelt, so dass dann der distale Endbereich 32 nach unten abgelenkt wird, wie das durch einen Pfeil angedeutet ist.

Entsprechend sind die Steuerseile 38 und 39 mit dem Steuerelement 23 verbunden. Wird dies im Uhrzeigersinn gedreht, wird das Zugseil 38 aufgewickelt und das Zugseil 39 abgewickelt und der distale Endbereich 32 wird, wie das in Fig. 1 mit durchgezogenen Linien dargestellt ist, nach rechts ausgelenkt. Dementsprechend verursacht dann eine Drehung des Steuerelementes 23 in die entgegengesetzte Richtung eine Ablenkung nach links, wie das durch die gestrichelten Linien dargestellt ist. Diese Bauteile stellen somit einen Auslenkmechanismus für den distalen Endbereich 32 dar.

Damit der ausgelenkte distale Endbereich 32 in einer bestimmten Auslenkposition verbleibt, ist ein Rastmechanismus vorgesehen, durch den der Auslenkmechanismus in dieser Stellung gehalten wird.

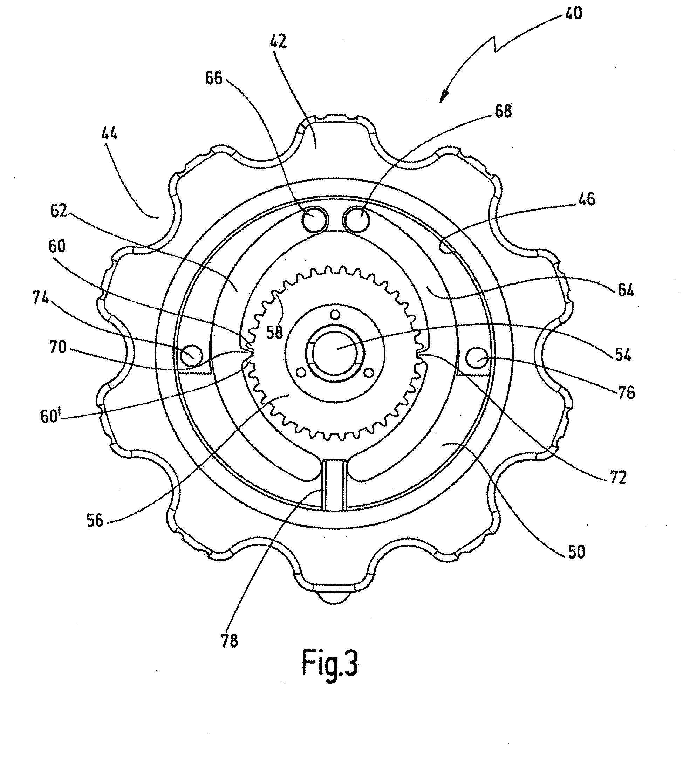

Das in Fig. 3 dargestellte Steuerelement 40 entspricht dem in Fig. 1 dargestellten ersten inneren Steuerelement 22 und beinhaltet einen solchen Rastmechanismus.

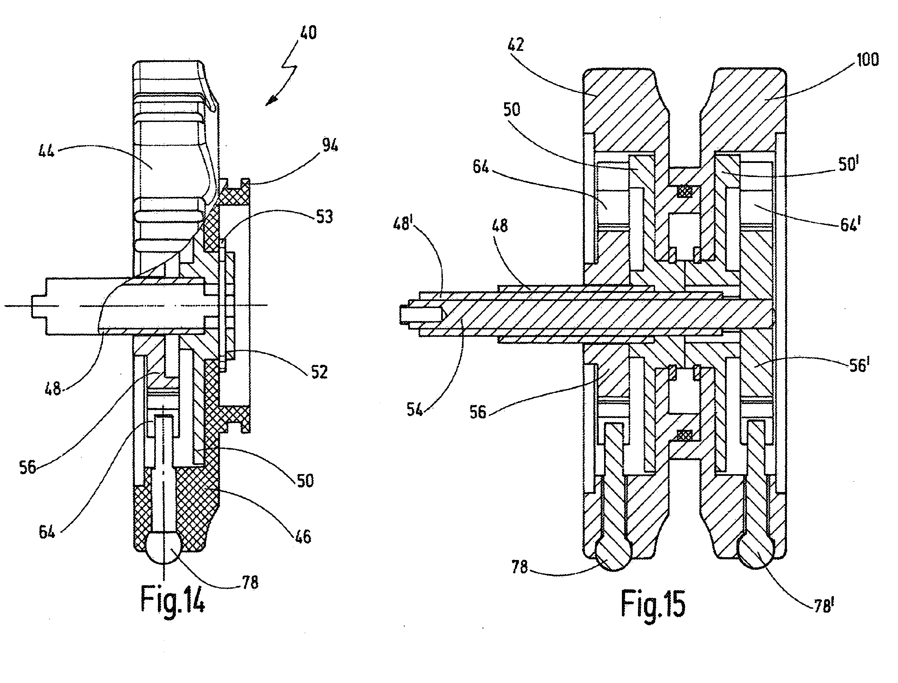

Wie aus Fig. 3 zu entnehmen, besteht das Steuerelement 40 in Form eines Drehrades 42, an dessen Außenseite mehrere Fingermulden 44 vorgesehen sind. Wie insbesondere auch aus der Teilschnittdarstellung von Fig. 14 zu erkennen, besteht das Steuerelement 40 aus einem Ringkörper 46.

Dieser Ringkörper 46 ist auf einen Montageflansch 52 einer Mitnehmerscheibe 50 aufgeschoben und dort über eine Sicherung 53 fest gehalten. Die Mitnehmerscheibe 50 selbst sitzt auf einer mittigen Welle 48. Durch die Welle 48 hindurch verläuft eine starre Achse 54, die ein Ritzel 56 trägt, das, in der Darstellung von Fig. 14 an dem linken, also dem Kopfstück 12 des Instruments 10 zugewandten Seite angeordnet ist. Die starre Achse 54 und somit auch das daran befestigte Ritzel 56 sind ebenfalls fest mit dem Kopfstück 12 des Instruments 10 unbeweglich verbunden.

Auf der dem Kopfstück 12 zugewandten Seite der Mitnehmerscheibe 50 sind zwei Klinken 62 und 64 montiert, wie das insbesondere aus Fig. 3 ersichtlich ist. Jede Klinke 62 und 64 besteht aus einem gebogenen Metallstreifen, die an von der Mitnehmerscheibe 50 vorstehenden Achszapfen 66 und 68 verschwenkbar angebracht sind. Von der Klinke 62 und ebenso spiegelbildlich gesehen von der Klinke 64 steht jeweils ein Klinkenzahn 70 bzw. 72 in Richtung der Zähne 60, 60' des Ritzels 56 vor.

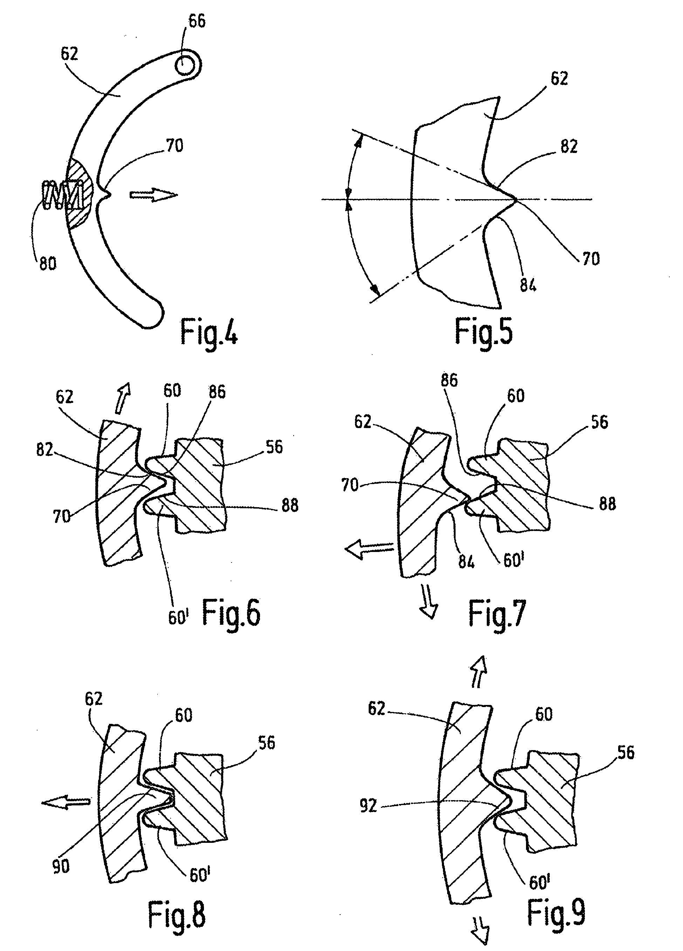

In Fig. 4 ist die Klinke 62 näher dargestellt. Daraus ist zu erkennen, dass in einer hier nicht näher bezeichneten Aussparung eine Feder 80 sitzt, deren eines Ende in der Ausnehmung aufgenommen ist und deren anderes Ende sich jeweils an einem Stift 74 bzw. 76 abstützt, der ebenfalls von der Mitnehmerscheibe 54 hochsteht.

Durch diese Federn 80 werden die Klinken 62 und 64 jeweils um die Achszapfen 66 bzw. 68 so verschwenkt, dass deren Klinkenzähne 70 bzw. 72 in das Ritzel also zwischen entsprechende Zähne 60, 60' der Reihe an Zähnen einrücken.

Aus der vergrößerten Darstellung von Fig. 5 ist zu erkennen, dass der Klinkenzahn 70 zwei unterschiedlich steile Flanken 82 bzw. 84 aufweist. Gegenüber der gestrichelten Mittellängsachse gesehen ist die Flanke 82 steiler als die Flanke 84.

Aus Fig. 6 ist zu erkennen, dass die Flanken 86 bzw. 88 der Zähne 60 bzw. 60' in etwa denen der Flanke 82 entsprechen.

Die Darstellung von Fig. 6 entspricht der Situation der Klinke 62 von Fig. 3.

Soll die Klinke 62 in Richtung des Pfeiles bewegt werden, wie er in Fig. 6 dargestellt ist, so sperrt die Flanke 82 des Klinkenzahnes 70 und die entsprechende Flanke 86 des Zahnes 60 diese Bewegung.

In Fig. 7 ist dargestellt, dass bei der entgegengesetzten Bewegung der Klinke 62, die flachere Flanke 84 längs der Flanke 88 des Zahnes 60' gleiten kann, wobei die Klinke 62 von den Zähnen 60, 60' weggerichtet bewegt wird.

In anderen Worten ausgedrückt, kann die Klinke 62 das Ritzel 56 in der in Fig. 7 mit dem Pfeil nach unten dargestellten Richtung überlaufen. Aufgrund der Kraft der Feder 80 bleibt aber die Klinke 62 ständig in Berührung mit dem Ritzel 56. Hat der Klinkenzahn 70 den Zahn 60' überfahren, rastet der Klinkenzahn 70 wieder in das Ritzel ein, so dass dann eine Situation wieder erreicht wird, wie sie in Fig. 6 dargestellt ist.

Aus der Bildfolge von Fig. 6 und 7 ist zu erkennen, dass eine Bewegung der Klinke 62 längs der Reihe 58 an Zähnen 60, 60' des Ritzel 56 in einer Richtung gesperrt ist, in der entgegengesetzten Richtung dieses aber überlaufen kann.

Aufgrund der Anlenkung an dem Achszapfen 66 kann der Klinkenzahn 70 in Richtung der Erhebung der Zähne 60, 60' aus dem Eingriff herausgewegt werden.

In Fig. 8 ist eine Situation dargestellt, bei der die Geometrie des Klinkenzahnes 90 so ausgebildet ist, dass dessen Flanken in etwa gleich konturiert sind wie die Flanken 86 und 88 der Zähne 60 und 60'. Bei dieser Ausgestaltung ist eine Bewegung der Klinke 62 in beiden Richtungen längs der Reihe an Zähnen gesperrt.

Diese Sperrung kann nur dann aufgehoben werden, wenn die Klinke 62 zuvor vollständig von den Zähnen 60 und 60' abgehoben ist.

In Fig. 9 ist eine Situation dargestellt, bei der der Klinkenzahn 92 so ausgebildet ist, dass er zwei relativ flache Flanken entsprechend der Flanke 84 aufweist.

In diesem Fall greift zwar der Klinkenzahn 92 zwischen die Zähne 60 und 60' des Ritzels ein und sperrt zunächst eine Längsbewegung zwischen Klinke 62 und Ritzel. Ist die Kraft, die auf die Klinke 62 einwirkt so stark, dass die Anpresskraft durch die Feder 80 überwunden wird, kann der Klinkenzahn 92 die Zähne 60 bzw. 60' des Ritzels 56 in beiden Richtungen überlaufen.

In allen Fällen ist durch ein Eintreten des entsprechenden Klinkenzahns 70, 90 oder 92 zwischen die Zähne 60, 60' eine Verriegelung oder Verrastung zwischen der Klinke 62 und dem Ritzel 56 bewerkstelligt.

In allen diesen Verrastungszuständen kann das Drehrad 40 nicht um das Ritzel 56 gedreht werden. Dazu muss der entsprechende Klinkenzahn, wie zuvor in Zusammenhang mit den Fig. 6 und 7 bzw. 8 oder 9 beschrieben, zwischen den Zähnen 60, 60' herausbewegt werden.

Ist der zuvor beschriebene Rastmechanismus zwischen Klinke 62 und Ritzel 56 gelöst, kann das Drehrad 42 weiter bewegt werden und somit über den Auslenkmechanismus dann der distale Endbereich 32 ausgelenkt werden.

In der Figurenfolge 10 bis 13 soll nunmehr die Ausführungsform des Rastmechanismus näher beschrieben werden, die zuvor in Zusammenhang mit den Fig. 4 bis 7 besprochen wurde und wie er prinzipiell in der Fig. 3 dargestellt ist.

Aus Fig. 3 ist eine Grundstellung erkennbar, in der der Schaft 30 geradlinig ausgerichtet ist. Beide Klinken 62 und 64 sind in das Ritzel 56 eingerastet. Die beiden bogenförmig gekrümmten Klinken 62 und 64 erstrecken sich von den Achszapfen 66 und 68 ausgehend bogenförmig beidseitig um das Ritzel 56 herum und liegen auf der den Achszapfen gegenüberliegenden Seiten beidseitig an einem sich radial von der Außenseite her durch den Ringkörper 46 des Drehrades 42 hindurchtretenden Mitnehmerstift 78 an.

Ein Drehen im Uhrzeigersinn ist deswegen gesperrt, da die steile Flanke 82 an der entsprechenden steilen Flanke 86 des Zahnes anliegt, wie das in Fig. 6 angedeutet ist. Aufgrund der spiegelbildlichen Ausbildung der Klinke 64 ist eine Bewegung entgegen dem Uhrzeigersinn gesperrt, da in dieser Bewegungsrichtung die steilere Flanke des Klinkenzahns 72 mit dem entsprechenden Zahn des Ritzels 56 in sperrendem Eingriff steht.

Da beide Klinken 62 und 64 durch die entsprechenden Federn 80 in Richtung auf das Ritzel 56 vorgespannt sind, hält diese Verriegelung ohne äußere Einwirkung. Anders ausgedrückt, wird dieser Zustand automatisch erreicht und gehalten.

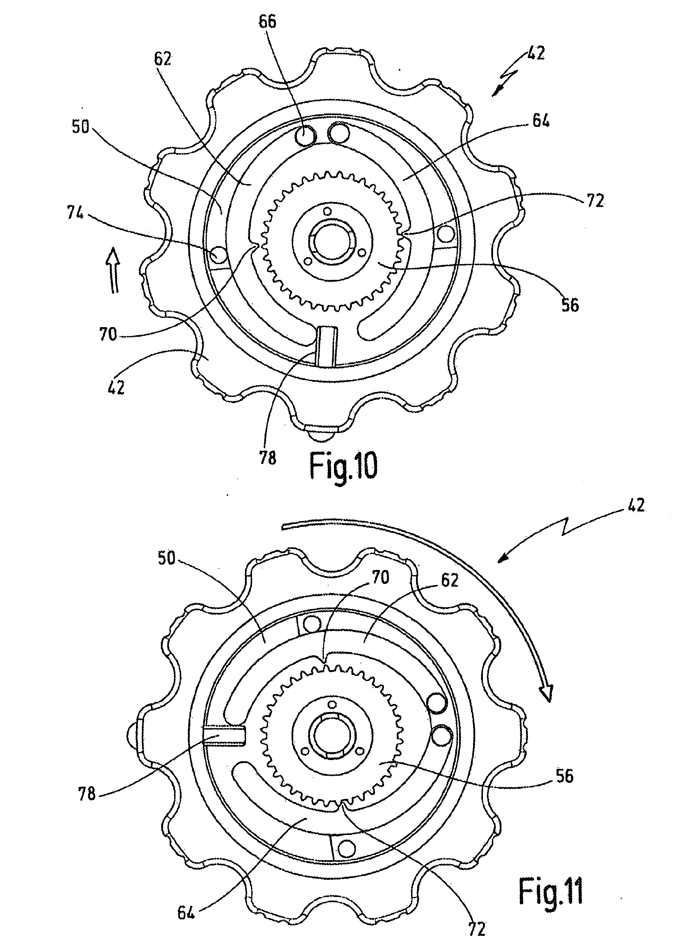

Wird nun das Drehrad 42 im Uhrzeigersinn etwas verdreht, wie das in Fig. 10 dargestellt ist, bewegt der Mitnehmerstift 78 die Klinke 62 vom Ritzel 56 weg, wodurch der sperrende Eingriff aufgehoben wird.

Die Klinke 62 kann soweit radial nach außen verschwenkt werden, bis diese auf den Stift 74 trifft und dort für einen Kraftschluss zwischen Drehrad 40, Mitnehmerstift 78, Klinke 62, Zapfen 74 und Mitnehmerscheibe 50 schließt.

Die Mitnehmerscheibe 50 dreht sich somit ab diesem Zeitpunkt ebenfalls im Uhrzeigersinn und bewegt dann die Steuerseile, wie zuvor beschrieben, es wird also der distale Endbereich des Schafts 30 ausgelenkt.

Wie aus Fig. 10 zu entnehmen, läuft der Klinkenzahn 72 der Klinke 64 in dieser Richtung über seine flache Flanke über die Zähne 60, 60' des Ritzels 56. Die hier nicht dargestellte Feder der Klinke 64 drückt dabei den Klinkenzahn 72 dauernd in Berührung mit dem Ritzel.

In Fig. 11 ist nunmehr eine Situation dargestellt, bei der das Drehrad 42 um etwa 90° im Uhrzeigersinn verdreht worden ist. Diese Verdrehung hat dann eine Verschwenkung des distalen Endbereichs 32 um 90° aus der geradlinigen Ausrichtung heraus verursacht.

Wird nun das Drehrad 42 in der aus Fig. 11 ersichtlichen Position freigegeben, drückt die Kraft der Feder 80 den Klinkenzahn 70 wieder in einen Freiraum zwischen zwei Zähne 60, 60' des Ritzels hinein. Dies erfolgt unmittelbar, ohne dass sich das Drehrad 42 weiter verdrehen kann, denn der Eingriff der Klinke 64 mit dem Ritzel 56 bietet schon in dieser Stelle eine Vorverrastung. Sollten in diesem Drehzustand gerade die Spitzen der Zähne von Klinke 62 und Ritzel 56 aufeinandertreffen, kann eine geringfügige Drehbewegung erfolgen, die aber keinen Einfluss mehr auf die Auslenkung ausübt. Ein Klemmen ist nicht möglich, da die Kraft der Klinke 62 nicht exakt radial einwirkt, sondern aufgrund deren Anlenkung mit einer tangentialen Komponente, so dass ein zwangsläufiges Einrasten der Klinke 62 erfolgt.

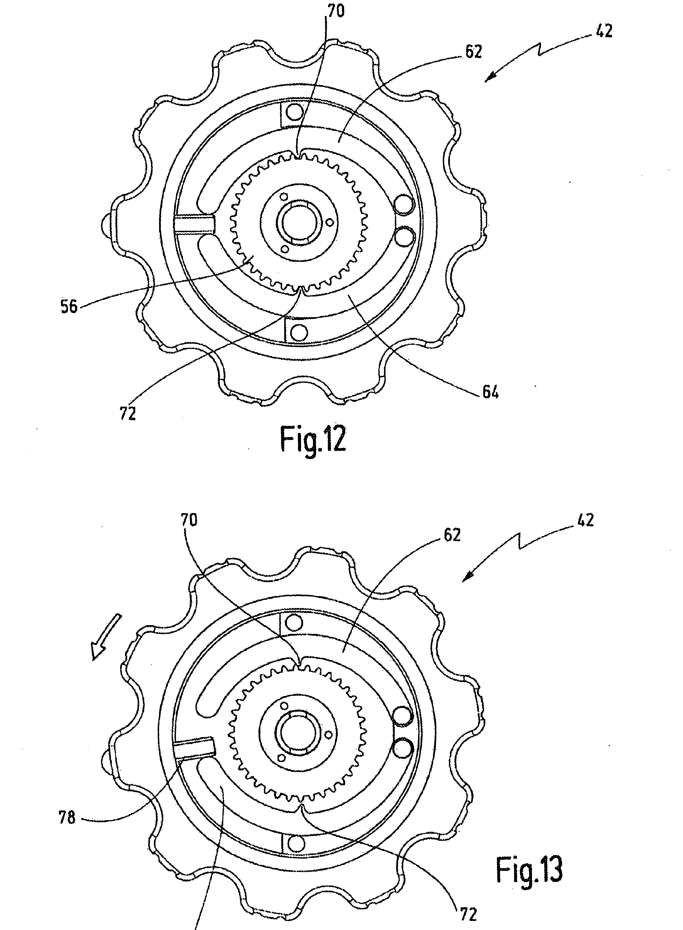

Dieser Zustand ist in Fig. 12 dargestellt.

Daraus ist ersichtlich, dass die Verriegelung durch den Rastmechanismus immer dann besteht und auch aufrechterhalten wird, wenn das Drehrad 42 freigegeben wird. Dies unabhängig von der Drehstellung, also beispielsweise in der Grundstellung von Fig. 3 oder in der Drehstellung von Fig. 12, bei der der distale Endbereich 32 ausgelenkt ist und erhebliche Rückstellkräfte auf das Drehrad 42 einwirken.

Soll der ausgelenkte distale Endbereich 32 wieder in die geradlinig ausgerichtete Position bewegt werden, wird das Drehrad 42 aus der in Fig. 12 beschriebenen Stellung entgegen dem Uhrzeigersinn verdreht.

Aus Fig. 13 ist zu entnehmen, dass dann der Mitnehmerstift 78 auf die andere gegenüberliegende Klinke 64 trifft, diese aus dem sperrenden Eingriff abhebt. In dieser Drehrichtung kann dann der Klinkenzahn 70 der Klinke 62 die Zähne des Ritzels 56 überfahren.

Ist dann beispielsweise der Endbereich 32 wieder geradlinig ausgerichtet, also die Position von Fig. 3 erreicht, kann das Drehrad 42 freigegeben, und beide Klinken 62 und 64 verrasten wieder.

Ein entsprechender Ablauf erfolgt, wenn beispielsweise das Drehrad 42 aus der in Fig. 3 dargestellten Stellung entgegen dem Uhrzeigersinn verdreht wird. Der Rastmechanismus kann somit als ein bidirektional selbstentriegelnder Rastmechanismus angesehen werden. Dieser Vorgang ist ergonomisch und auch haptisch sehr angenehm durchzuführen, da das Drehrad 42 über die Fingermulden 44 sicher ergriffen werden kann, und Manipulationen nur in einer bestimmten Drehrichtung unter Verdrehen des Drehrades 42 durchgeführt werden müssen, so dass keinerlei Aufmerksamkeit dahingehend erbracht werden muss, ob eine Verriegelung oder Verrastung geöffnet oder geschlossen ist, da diese automatisch schließt, wenn das Drehrad 42 freigegeben wird bzw. zwangsläufig entrastet wird, sobald das Drehrad 42 gedreht wird.

In Fig. 14 ist dargestellt, wie das zuvor beschriebene Steuerelement 40 montiert ist.

Eingänglich wurde beschrieben, dass bei manchen Ausführungen eine Verschwenkung des distalen Endbereiches 32 des Schaftes 30 in zwei verschiedenen Ebenen erwünscht wird.

Dazu ist, wie das aus Fig. 15 ersichtlich, auf das erste Steuerelement 40 bzw. das Drehrad 42 ein zweites entsprechendes etwa spiegelbildlich ausgebildetes Drehrad 100 montiert. Dazu steht von dem Drehrad 40, wie das aus Fig. 14 ersichtlich ist, ein Montageflansch 94 vor, auf dem das Drehrad 100 aufgeschoben und gehalten werden kann, wie das in Fig. 15 ersichtlich ist. Das so aufgeschobene Drehrad 100 ist gegen Abfallen gesichert, ist aber unabhängig vom Drehrad 42 drehbar.

In diesem Falle erstreckt sich die mittige starre Achse 54 soweit, dass sie sich bis in das zweite Drehrad 100 hineinerstreckt, so dass auf diesem auch das Ritzel 56' dieses Drehrades montiert werden kann. Auch hier ist dann wieder eine entsprechende Mitnehmerscheibe 50' vorgesehen, auf der entsprechende Klinken montiert sind, wobei hier die Klinke 64' ersichtlich ist.

Auch hier ist wieder ein Mitnehmerstift 78' vorgesehen, der sich zwischen die beiden Klinken hineinerstreckt und je nach Drehrichtung des Drehrades 100 diese abhebt und somit die Verriegelung aufhebt. Das Drehrad 100 ist auf einer weiteren Welle 48' montiert, so dass dann, nach Kraftschluss der entsprechend ausgelenkten Klinke des Drehrades 100 diese Welle 48' gedreht wird. Aus Fig. 15 ist zu entnehmen, dass diese Welle 48' innerhalb der Welle 48 des Drehrades 42 verläuft und über die Außenseite der starren Achse 54 läuft. Somit kann dann durch die Welle 48' das entsprechend andere Steuerseilpaar bewegt bzw. gesteuert werden.

Wird die in Fig. 9 dargestellte Zahngeometrie gewählt, ist bei durch den Mitnehmerstift 78 abgehobener einer Klinke ein entsprechender Anpressdruck der gegenüberliegenden anderen Klinke vorzusehen, so dass diese permanent mit dem Ritzel in Berührung steht.

Diese Konstruktion kann man wählen, wenn die Rückstellkräfte durch den ausgelenkten Endbereich 32 nicht sehr groß sind.

Sollten diese Rückstellkräfte extrem groß sein, oder wirken auf den ausgelenkten distalen Endbereich aufgrund von Manipulationen extreme Rückstellkräfte ein, kann man eine Zahnform wie aus Fig. 8 in Erwägung ziehen.

Dann muss man allerdings sicherstellen, dass bei einem Drehen des Drehrades beide Klinken abgehoben werden.

Es wurden bislang immer zwei Klinken als separate Bauteile beschrieben.

Diese können aus Federstahl ausgebildet sein und durch entsprechende Montage gespannt am Ritzel anliegen. Dann kann auf die Federn 80 verzichtet werden.

Beide Klinken können als einstückiges Bauteil hergestellt werden, die im Bereich der Achszapfen 66 und 68 miteinander verbunden sind.

Das Grundprinzip muss immer erfüllt werden, d.h. dass nach Freigabe des Drehrades der Rastmechanismus automatisch schließt, dieser aber mit ein und derselben Bewegung des Drehrades wie bei dem Bewegen des Auslenkmechanismus gelöst wird.

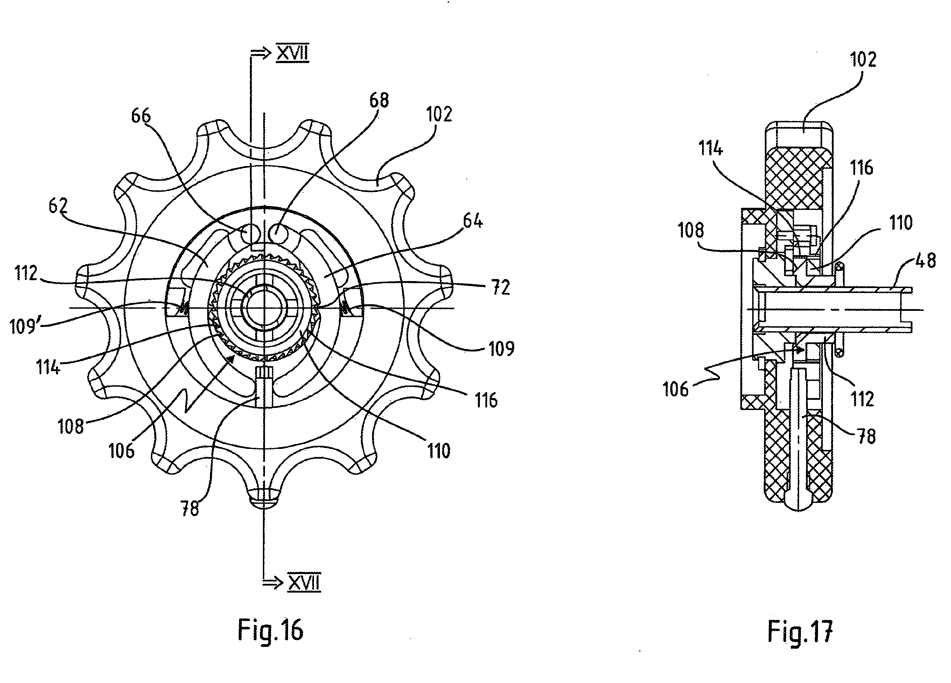

In den Fig. 16 und 17 ist ein weiteres Ausführungsbeispiel eines Steuerelementes in Form eines Drehrades 102 dargestellt. Für gleiche bzw. identische Bauelemente, wie sie in Zusammenhang mit den zuvor beschriebenen Ausführungsbeispielen erwähnt wurden, werden dieselben Bezugsziffern verwendet.

Auch das Drehrad 102 ist, wie in Zusammenhang mit Fig. 3 beschrieben, als Ringkörper aufgebaut, der auf eine Mitnehmerscheibe aufgeschoben ist und dort über eine Sicherung fest gehalten ist. Auf einer mittigen Welle 48 ist ein Ritzel 106 montiert, das aus zwei aufeinander gesetzten Ritzelscheiben 108 und 110 aufgebaut ist.

Dabei ist die innere, oder in der Darstellung von Fig. 16 untere erste Ritzelscheibe 108 mit einem hochstehenden Flansch 112 vorgesehen, auf den die zweite Ritzelscheibe 110 aufgeschoben ist.

Die erste Ritzelscheibe 108 trägt an ihrer äußeren Umfangskante eine erste Reihe an Zähnen 114, die asymmetrisch ausgebildet sind.

Die zweite Ritzelscheibe 110 trägt an ihrer äußeren Umfangskante eine zweite Reihe an Zähnen 116, die ebenfalls asymmetrisch, aber in entgegengesetzter Richtung ausgerichtet sind.

Die erste Reihe an Zähnen 114 der ersten Ritzelscheibe 108 steht mit einem hier nicht näher bezeichneten Klinkenzahn der ersten Klinke 62 in Eingriff.

Die zweite Reihe an Zähnen 116 der zweiten Ritzelscheibe 110 steht mit dem entsprechenden asymmetrischen Klinkenzahn 72 der zweiten Klinke 64 in Eingriff.

Das heißt, die beiden Klinken 62 und 64 sind auf unterschiedlicher Höhe montiert, damit deren Klinkenzahn jeweils mit der diesem zugeordneten Reihe an Zähnen 114 bzw. 116 in Eingriff treten kann.

Wie aus Fig. 16 zu entnehmen, ist der asymmetrische Klinkenzahn 72 der zweiten Klinke 64 so ausgebildet, dass er in Uhrzeigerrichtung gesehen die Zähne 116 überlaufen kann, entgegen dem Uhrzeigersinn aber sperrt. Eine Feder 109 sorgt dafür, dass die zweite Klinke 64 an die Zähne 116 angedrückt wird. Entsprechendes gilt für die gegenüberliegende erste Klinke 62, die ebenfalls durch eine entsprechende Feder 109' an die in der Darstellung von Fig. 16 darunter gelegene Reihe an Zähnen 114 angedrückt wird. Die zweite Klinke 64 sperrt in entgegengesetzter Richtung zur ersten Klinke 62.

Auch hier ist das Funktionsprinzip gleich, d.h. wird das Drehrad 102 beispielsweise entgegen dem Uhrzeigersinn bewegt, hebt der Mitnehmerstift 78 die zweite Klinke 64 bzw. den Klinkenzahn 72 vom sperrenden Eingriff von der Reihe an Zähnen 116 gegen die Kraft der Feder 109 ab. Dies erfolgt nahezu spielfrei. Nunmehr kann das Drehrad 102 entgegen dem Uhrzeigersinn verdreht werden. Dabei überläuft die erste Klinke 62 die Reihe an Zähnen 114, und zwar gegen die Kraft der Feder 109'.

Wird das Drehrad 102 freigegeben, drückt die Feder 109 den Klinkenzahn 72 der zweiten Klinke 64 in die ihm nächst angebotene Lücke zwischen zwei benachbarten Zähnen 116 und verrastet den Mechanismus wieder.

Entsprechendes gilt für die entgegengesetzte Drehrichtung. Dabei hebt der Mitnehmerstift 78 die erste Klinke 62 von der "unteren" Reihe an Zähnen 114 ab und der Klinkenzahn 72 der zweiten Klinke 64 überläuft die Zähne 116 der zweiten Ritzelscheibe 110.

## Patentansprüche

1. Instrument, mit einem proximalseitigen Kopfstück (12), mit einem zumindest im distalen Endbereich (32) über einen Auslenkme chanismus auslenkbaren Schaft (30), mit einem Steuerelement (22, 23, 40), durch das Auslenkbewegungen des Schaftes (30) durch Bewegen von Steuerseilen (36, 37; 38, 39) des Auslenkmechanismus steuerbar sind, und mit einer Sperre zum Sperren des Auslenkmechanismus, **dadurch gekennzeichnet, dass** die Sperre einen Rastmechanismus aufweist, der derart mit dem Steuerelement (40) verbunden ist, dass der Rastmechanismus, ohne Betätigung des Steuerelements (40), zwangsläufig in eine sperrende Stellung läuft, und dass eine Bewegung des Steuerelements (40) in eine Richtung zunächst eine Öffnung des Rastmechanismus bewirkt und erst dann eine Auslenkung des Schaftes (30) ermöglicht, und dass eine Freigabe des Steuerelements (40) in irgendeiner Stellung des Auslenkmechanismus (27) eine zwangsläufige Sperrung durch den Rastmechanismus in dieser Stellung verursacht.

2. Instrument nach Anspruch 1, **dadurch gekennzeichnet, dass** der Rastmechanismus eine Reihe (58) an Zähnen (60, 60'; 114,116) aufweist, in die eine bewegbare Klinke (62, 64) zwangsläufig sperrend einfahrbar ist, die vom Steuerelement (40) aus dem sperrenden Eingriff heraus bewegbar ist.

3. Instrument nach Anspruch 2, **dadurch gekennzeichnet, dass** die Klinke (62, 64) federelastisch (80) in Richtung der Reihe (58) an Zähnen (60, 60'; 114,116) vorgespannt ist.

4. Instrument nach Anspruch 2 oder 3, **dadurch gekennzeichnet, dass** die Reihe (58) an Zähnen (60, 60'; 114,116) und die Klinke (62, 64) derart geformt sind, dass eine Bewegung der Klinke (62, 64) längs der Reihe (58) gesperrt ist, und erst eine Bewegung der Klinke (62, 64) aus der Reihe (58) heraus eine Bewegung ermöglicht.

5. Instrument nach einem der Ansprüche 2 bis 4, **dadurch gekennzeichnet, dass** die Reihe (58) an Zähnen (60, 60') und die Klinke (62, 64) derart geformt sind, dass unter Überwindung einer vorgegebenen Gegenkraft die Klinke (62, 64) längs der Reihe an Zähnen bewegbar ist.

6. Instrument nach einem der Ansprüche 2 bis 5, **dadurch gekennzeichnet, dass** die Reihe (58) an Zähnen (60, 60') und die Klinke (62, 64) derart geformt sind, dass eine Bewegung der Klinke (62, 64; 114,116) in einer Richtung der Reihe (58) gesperrt und in der entgegengesetzten Richtung unter Überwindung einer Gegenkraft möglich ist.

7. Instrument nach Anspruch 6, **dadurch gekennzeichnet, dass** die Klinke (62, 64) einen asymmetrischen Klinkenzahn (70, 72) aufweist, der die Bewegung der Klinke (62) oder (64) in der einen Richtung durch Formschluss sperrt und in der anderen entgegengesetzten Richtung unter Überwindung einer Gegenkraft die Reihe an Zähnen (60, 60'; 114,116) überlaufen kann.

8. Instrument nach einem der Ansprüche 2 bis 7, **dadurch gekennzeichnet, dass** zwei Klinken (62, 64) vorhanden sind, wobei eine erste Klinke in einer ersten Richtung längs der Reihe an Zähnen (60, 60'; 114,116) sperrt und die zweite Klinke in einer zweiten, der ersten entgegengesetzten Richtung sperrt, wobei das Steuerelement (40) bei Bewegen in der ersten Richtung die erste Klinke aus dem sperrenden Eingriff löst und bei Bewegen in der zweiten Richtung die zweite Klinke löst.

9. Instrument nach Anspruch 8, **dadurch gekennzeichnet, dass** die jeweils andere Klinke, die nicht vom Steuerelement (40) aus dem sperrenden Eingriff bewegt ist, unter Überwindung einer Anpresskraft die Reihe (58) an Zähnen (60, 60'; 114,116) überlaufen kann.

10. Instrument nach einem der Ansprüche 2 bis 9, **dadurch gekennzeichnet, dass** die Reihe (58) an Zähnen (60, 60') als Ritzel (56; 106) ausgebildet ist, um das die Klinke bzw. die Klinken (62, 64) als schwenkbare einarmige Hebel angeordnet ist bzw. sind.

11. Instrument nach Anspruch 10, **dadurch gekennzeichnet, dass** das Ritzel (106) zwei Reihen an Zähnen (114, 116) aufweist, wobei die Zähne (114) einer ersten Reihe mit der ersten Klinke (62) und die Zähne (116) einer zweiten Reihe mit der zweiten Klinke (64) in Eingriff bringbar sind.

12. Instrument nach Anspruch 11, **dadurch gekennzeichnet, dass** das Ritzel (106) aus zwei übereinander gesetzten Ritzelscheiben (108, 110) aufgebaut ist, die die jeweilige Reihe an entgegengesetzt gerichteter Zähne (114 bzw. 116) aufweisen.

13. Instrument nach einem der Ansprüche 10 bis 12, **dadurch gekennzeichnet, dass** das Steuerelement (40) als Drehrad (42) ausgebildet ist, das um das ortsfest angeordnete Ritzel (56, 106) drehbar ist.

14. Instrument nach Anspruch 13, **dadurch gekennzeichnet, dass** am Drehrad (42) ein Mitnehmer (78) angeordnet ist, der in jeder Drehrichtung mit einer Klinke (62 oder 64) in Eingriff steht und diese dabei aus dem sperrenden Eingriff bringt und so hält, und zwar solange, wie das Drehrad (42) die dazu erforderliche Kraft auf die Klinke ausübt.

15. Instrument nach Anspruch 14, **dadurch gekennzeichnet, dass** die ausgerastete Klinke vom Mitnehmer (78) gegen einen Anschlag an einer Mitnehmerscheibe (50) gedrückt wird, wobei die Mitnehmerscheibe (50) kraftschlüssig mit dem Auslenkmechanismus in Verbindung steht.

16. Instrument nach einem der Ansprüche 1 bis 15 mit einem Schaft (30), der unabhängig in zwei Ebenen auslenkbar ist, **dadurch gekennzeichnet, dass** er zwei derart ausgestaltete Auslenkmechanismen aufweist.

17. Instrument nach Anspruch 16, **dadurch gekennzeichnet, dass** zwei aufeinander gesetzte Drehräder (42, 100) vorgesehen sind, die mechanisch aneinander gehalten sind, jedoch eine Betätigung jedes der Drehräder unabhängig (42, 100) vom anderen ermöglichen.

## Claims

1. Instrument with a proximal headpiece (12), with a shaft (30) that can be deflected at least in the distal end area (32) via a deflection mechanism, with a control element (22, 23, 40) via which deflection movements of the shaft (30) can be controlled by movement of control wires (36, 37; 38, 39) of the deflection mechanism, and with a lock for locking the deflection mechanism, **characterized in that** the lock has a catch mechanism which is connected to the control element (40) in such a way that the catch mechanism, without actuation of the control element (40), is forced into a locking position, and **in that** a movement of the control element (40) in one direction first opens the catch mechanism and only then permits a deflection of the shaft (30), and **in that** release of the control element (40), in any position of the deflection mechanism, causes an enforced locking by the catch mechanism in this position.

2. Instrument of Claim 1, **characterized in that** the catch mechanism has a row (58) of teeth (60, 60'; 114, 116) into which a movable detent (62, 64) can be introduced with a positive locking action, which detent (62, 64) can be moved out of the locking engagement by the control element (40).

3. Instrument of Claim 2, **characterized in that** the detent (62, 64) is pretensioned resiliently (80) in the direction of the row (58) of teeth (60, 60'; 114, 116).

4. Instrument of Claims 2 or 3, **characterized in that** the row (58) of teeth (60, 60'; 114, 116) and the detent (62, 64) are formed in such a way that a movement of the detent (62, 64) along the row (58) is blocked, and only a movement of the detent (62, 64) out of the row (58) permits a movement.

5. Instrument of anyone of Claims 2 to 4, **characterized in that** the row (58) of teeth (60, 60') and the detent (62, 64) are formed in such a way that the detent (62, 64) can be moved along the row of teeth when a predetermined counter-force is overcome.

6. Instrument of anyone of Claims 2 to 5, **characterized in that** the row (58) of teeth (60, 60') and the detent (62, 64) are formed in such a way that a movement of the detent (62, 64) is blocked in one direction of the row (58) and is possible in the opposite direction when a counter-force is overcome.

7. Instrument of Claim 6, **characterized in that** the detent (62, 64) has an asymmetrical detent tooth (70, 72) which blocks the movement of the detent (62) or (64) in one direction by positive locking and which can run over the row of teeth (60, 60'; 114, 116) in the other, opposite direction, when a counter-force is overcome.

8. Instrument of anyone of Claims 2 to 7, **characterized in that** two detents (62, 64) are present, of which a first detent provides locking in a first direction along the row of teeth (60, 60'; 114, 116), and of which the second detent provides locking in a second direction opposite to the first direction, and the control element (40), upon movement in the first direction, releases the first detent from the locking engagement and, upon movement in the second direction, releases the second detent.

9. Instrument of Claim 8, **characterized in that** the respective other detent, which is not moved out of the locking engagement by the control element (40), can run over the row (58) of teeth (60, 60'; 114, 116) when a pressing force is overcome.

10. Instrument of anyone of Claims 2 to 9, **characterized in that** the row (58) of teeth (60, 60'; 114, 116) is designed as a pinion (56; 106) around which the detent or detents (62, 64) is or are arranged as a pivotable single-arm lever.

11. Instrument of Claim 10, **characterized in that** the pinion (106) has two rows of teeth (114, 116), wherein teeth (114) of a first row can be brought in engagement with the first detent (62) and wherein teeth (116) of a second row can be brought in engagement with the second detent (64).

12. Instrument of Claim 11, **characterized in that** the pinion (106) is assembled of two superposed pinion disks (108, 110) having the respective row of oppositely directed teeth (114, 116).

13. Instrument of anyone of Claims 10 to 12, **characterized in that** the control element (40) is designed as a rotary wheel (42), which is rotatable about the stationary pinion (56; 106).

14. Instrument of Claim 13, **characterized in that** a driver (78) is arranged on the rotary wheel (42), which driver (78), in each direction of rotation, engages with a detent (62 or 64) and in so doing releases it from the locking engagement and keeps it released for as long as the rotary wheel (42) exerts the force required for this on the detent.

15. Instrument of Claim 14, **characterized in that** the released detent is pressed by the driver (78) against an abutment on a driver disc (50), said driver disc (50) being connected with a force fit to the deflection mechanism.

16. Instrument of anyone of Claims 1 to 15, with a shaft (30) that can be deflected independently in two planes, **characterized in that** it has two such deflection mechanisms.

17. Instrument of Claim 16, **characterized in that** two rotary wheels (42, 100) are provided which are placed on each other and are held mechanically on each other, but which permit actuation of each of the rotary wheels (42, 100) independently of the other.

## Revendications

1. Instrument avec une pièce de tête (12) proximale, avec au moins un corps (30) pouvant être dévié au moins dans la zone d'extrémité distale (32) par l'intermédiaire d'un mécanisme de déviation, avec un élément de commande (22, 23, 40) au moyen duquel les mouvements de déviation du corps (30) peuvent être commandés par déplacement de câbles de commande (36, 37 ; 38, 39) du mécanisme de déviation, et avec un mécanisme de blocage pour bloquer le mécanisme de déviation, **caractérisé en ce que** le mécanisme de blocage présente un mécanisme d'encliquetage qui est relié à l'élément de commande (40) de façon que le mécanisme d'encliquetage, en l'absence d'actionnement de l'élément de commande (40), aille obligatoirement dans une position de blocage, et qu'un déplacement de l'élément de commande (40) dans un sens entraîne d'abord une ouverture du mécanisme d'encliquetage et permette ensuite seulement une déviation du corps (30), et qu'un déblocage de l'élément de commande (40) dans une position quelconque du mécanisme de déviation (27) provoque un blocage obligatoire dans cette position par le mécanisme d'encliquetage.

2. Instrument selon la revendication 1, **caractérisé en ce que** le mécanisme d'encliquetage présente une série (58) de dents (60, 60' ; 114, 116) dans laquelle un cliquet mobile (62, 64) peut être engagé de manière à obtenir un blocage obligatoire, lequel cliquet peut être sorti de l'engagement bloquant par l'élément de commande (40).

3. Instrument selon la revendication 2, **caractérisé en ce que** le cliquet (62, 64) est précontraint par un élément élastique (80) en direction de la série (58) de dents (60, 60' ; 114, 116).

4. Instrument selon la revendication 2 ou 3, **caractérisé en ce que** la série (58) de dents (60, 60' ; 114, 116) et le cliquet (62, 64) sont formés de façon qu'un déplacement du cliquet (62, 64) le long de la série (58) soit bloqué et que seul un déplacement du cliquet (62, 64) pour sortir de la série (58) permette un déplacement.

5. Instrument selon une des revendications 2 à 4, **caractérisé en ce que** la série (58) de dents (60, 60') et le cliquet (62, 64) sont formés de façon que le cliquet (62, 64) puisse être déplacé le long de la série de dents en surmontant une force antagoniste prédéfinie.

6. Instrument selon une des revendications 2 à 5, **caractérisé en ce que** la série (58) de dents (60, 60') et le cliquet (62, 64) sont formés de façon qu'un déplacement du cliquet (62, 64 ; 114, 116) soit bloqué dans un sens de la série (58) et qu'il soit possible dans le sens opposé en surmontant une force antagoniste.

7. Instrument selon la revendication 6, **caractérisé en ce que** le cliquet (62, 64) présente une dent de cliquet asymétrique (70, 72) qui bloque le déplacement du cliquet (62) ou (64) par engagement positif dans un sens et peut passer par-dessus la série de dents (60, 60' ; 114, 116) dans l'autre sens, opposé, en surmontant une force antagoniste.

8. Instrument selon une des revendications 2 à 7, **caractérisé en ce que** deux cliquets sont prévus (62, 64), un premier cliquet bloquant dans un premier sens le long de la série de dents (60, 60' ; 114, 116) et le second cliquet bloquant dans un second sens, opposé au premier, l'élément de commande (40) libérant le premier cliquet de l'engagement bloquant lors du déplacement dans le premier sens et libérant le second cliquet lors du déplacement dans le second sens.

9. Instrument selon la revendication 8, **caractérisé en ce que** l'autre cliquet respectif, qui n'est pas sorti de l'engagement bloquant par l'élément de commande (40), peut passer par-dessus la série de dents (60, 60' ; 114, 116) en surmontant une force d'application.

10. Instrument selon une des revendications 2 à 9, **caractérisé en ce que** la série (58) de dents (60, 60') est réalisée sous la forme d'un pignon (56 ; 106) autour duquel le cliquet ou les cliquets (62, 64) est ou sont disposé(s) sous la forme de leviers à bras unique pivotants.

11. Instrument selon la revendication 10, **caractérisé en ce que** le pignon (106) présente deux séries de dents (114, 116), les dents (114) d'une première série pouvant être mises en prise avec le premier cliquet (62) et les dents (116) d'une seconde série avec le second cliquet (64).

12. Instrument selon la revendication 11, **caractérisé en ce que** le pignon (106) est constitué de deux disques de pignon (108, 110) posés l'un sur l'autre qui présentent des séries de dents (114, 116) orientées en sens contraire.

13. Instrument selon une des revendications 10 à 12, **caractérisé en ce que** l'élément de commande (40) est réalisé sous la forme d'une molette (42) qui peut tourner autour du pignon (56, 106) disposé en position fixe.

14. Instrument selon la revendication 13, **caractérisé en ce qu'**un élément d'entraînement (78) est disposé sur la molette (42), lequel est en prise avec un cliquet (62 ou 64) dans chaque sens de rotation et fait sortir celui-ci de l'engagement bloquant et le maintient dans cet état aussi longtemps que la molette (42) exerce la force nécessaire à cet effet sur le cliquet.

15. Instrument selon la revendication 14, **caractérisé en ce que** le cliquet décliqueté de l'élément d'entraînement (78) est pressé contre une butée sur un disque d'entraînement (50), le disque d'entraînement (50) étant relié par force au mécanisme de déviation.

16. Instrument selon une des revendications 1 à 15 avec un corps (30) qui peut être dévié de façon indépendante dans deux plans, **caractérisé en ce qu'**il présente deux mécanismes de déviation réalisés de cette manière.

17. Instrument selon la revendication 16, **caractérisé en ce que** deux molettes (42, 100) posées l'une sur l'autre sont prévues, qui sont tenues ensemble mécaniquement mais permettent néanmoins un actionnement de chacune des molettes (42, 100) de façon indépendante de l'autre.
